# EUROPEAN PATENT SPECIFICATION

(11) **EP 0 378 173 B1**
(45) Date of publication and mention of the grant of the patent: **23.11.1994**
(21) Application number: 90100376.4
(22) Date of filing: 09.01.1990
(51) Int. Cl.: G01N 33/53, G01N 33/542, G01N 33/577, G01N 33/22

(54) **A method for the detection and analysis of organic nitro compounds**
Ein Verfahren zum Nachweis und zur Analyse von organischen Nitroverbindungen
Une méthode pour la détection et l'analyse de composés nitroorganique

(30) Priority: 09.01.1989 JP 2423/89
(43) Date of publication of application: 18.07.1990
(73) Proprietor: MATSUSHITA ELECTRIC INDUSTRIAL CO., LTD., Kadoma-shi, Osaka-fu, 571 (JP)
(72) Inventor: Sugihara, Hirokazo, Higashiosaka-shi, Osaka (JP); Mitsumata, Tadayasu, Hirakata-shi, Osaka (JP); Miyazaki, Jinsei, D-3400 Göttingen (DE)
(74) Representative: Schwabe - Sandmair - Marx

(56) References cited:
- CHEMICAL ABSTRACTS, vol. 93, no. 19, 10 November 1980, Columbus, OH (US); P. GETTINS et al., p. 478, no. 184107q#
- CHEMICAL ABSTRACTS, vol. 113, no. 3, 16 July 1990, Columbus, OH (US); T. MITSUMATA et al., p. 319, no. 20423v#
- CHEMICAL ABSTRACTS, vol. 111, no. 25, 18 December 1989, Columbus, OH (US); p. 257, no. 227113y#
- CHEMICAL ABSTRACTS, vol. 112, no. 21, 21 May 1990, Columbus, OH (US); p. 255, no. 193659j#

## Description

### 1. Field of the invention:

This invention relates to a method for the detection and analysis of organic nitro compounds present in trace amounts in gas samples. The gas sample is most typically air, but other gases besides air can be detected as well.

By this method for detection and analysis, it is possible to detect organic nitro compounds present in the air in trace amounts, the nitro compounds being discharged from an explosive or from a gun or other firearm that uses organic nitro compounds as an explosive compound, so that the existence of such an explosive and firearm can be discovered. Thus, the method for the detection and analysis of organic nitro compounds of this invention is useful for the control of the illegal importation of explosives, firearms, and the like, and for the control of the illegal transportation of such in public transportation facilities such as trains, boats and ships, airplane, and airports. It should be effective in the control and prevention of terrorist acts and other dangerous occurrences. In this way, the method of this invention will be able to contribute greatly to increased safety in society.

### 2. Description of the prior art:

Methods and apparatus that have been used in the detection of explosives in cargo, hand luggage articles to be mailed, and the like have included mass spectrometers, gas chromatography apparatus, X-ray devices, etc.

Of these devices, mass spectrometers and gas chromatography apparatus are used in the detection of the explosives themselves or of firearms that have discharged live ammunition by the detection of organic nitro compounds. X-ray devices are used in the detection of metallic objects, and are mainly useful in the detection of arms made of metal. However, even with the use of these available methods and devices, the detection of explosives and firearms has been very difficult.

When mass spectrometry or gas chromatography is used, the detection limit of organic nitro compounds is no better than about 10⁻⁸ g/ml, so these methods are not satisfactory for the detection in the air of trace amounts of organic nitro compounds. Also, the time required for a single measurement is from 20 minutes to 5 hours, which is very long, and these methods therefore cannot be used at all at airports and the like where rapid inspection of hand luggage is necessary.

In addition, these devices cannot detect organic nitro compounds specifically. For that reason, when these devices are used for the measurement of organic nitro compounds in the air, the various other components in the air cause interference, and it becomes more difficult to detect the organic nitro compounds.

X-ray devices are hazardous to handle, because a means to generate X rays is incorporated inside them. In addition, when firearms and the like are to be detected in cargo and hand luggage, which contain many articles made of a variety of materials, if the firearms, etc., are to be detected speedily and accurately, the operator must be skillful.

The detection of organic nitro compounds could also be of use when immunochemical procedures are being done, although this is not now one of its uses. As typical methods for the detection of substances by the use of immunochemistry, a variety of enzyme immunoassays have been established. As a rule, in enzyme immunoassays, measurement can be more sensitive than with the devices for measurement mentioned above. Also, because an antibody that binds specifically to the organic nitro compounds that are the target of the measurement is used, organic nitro compounds can be detected without interference by other components present in the air.

However, the procedures used in enzyme immunoassays are complicated, and if results are to have satisfactory reproducibility, the operator must be skillful. The time needed for measurement is from several tens of minutes to several days, and rapid measurement is difficult.

### SUMMARY OF THE INVENTION

The method for the detection and analysis of organic nitro compounds of this invention, which overcomes the above-discussed and numerous other disadvantages and deficiencies of the prior art, comprises mixing a sample solution with a solution of antibody that binds specifically to said organic nitro compounds dissolved in solvent so as to prepare a mixed solution, and measuring the fluorescence intensity of said mixed solution to thereby detect the presence and/or the amount of said organic nitro compounds.

In a preferred embodiment, the mixture of the sample solution and of the antibody solution to prepare a mixed solution and also the measurement of the fluorescence of said mixed solution are carried out continuously in a flow system.

In a preferred embodiment, a sample gas is dissolved in solvent for the preparation of said sample solution.

In a preferred embodiment, the antibody is a monoclonal antibody.

In a preferred embodiment, the organic nitro compound to be detected is an aromatic nitro compound.

In a preferred embodiment, the organic nitro compound to be detected is one selected from the group consisting of trinitrotoluene (TNT), hexogen, octogen, nitroglycerin, nitrocellulose, nitroglycol, and penthrite.

Thus, the invention described herein makes possible the objectives of (1) providing a method for the detection and analysis of organic nitro compounds that has a higher sensitivity than the conventional instruments for analysis mentioned above, with high speed, and with specificity for organic nitro compounds; and (2) providing a method for the detection and analysis of organic nitro compounds that does not need skill for detection, is excellent in reproducibility, and involves a simple procedure.

This invention makes use of the fluorescence quenching properties of organic nitro compounds in the detection of these compounds, in order to overcome the problems described above.

By the introduction of a gas sample to be tested into a solvent while it is being bubbled through the solvent, the components that are soluble in the solvent are dissolved in the solvent, which becomes a sample solution for testing. An antibody that binds specifically to the substances (i.e., organic nitro compounds) that are to be detected is then dissolved in the solvent, after which the sample solution is mixed with the solution of the antibody. If trace amounts of the organic nitro compounds are contained in the gas to be tested, when the two solutions are mixed, an antigen-antibody reaction will occur in less than one second, with the binding of the organic nitro compounds and the antibody. At this time, the intensity of the fluorescence of the free antibody will decrease as binding with the organic nitro compounds occurs, and fluorescence quenching will take place. The fluorescence quenching phenomenon can be observed on a number of antibodies that bind to organic nitro compounds. By the calculation of the percentage of decrease in the intensity of the fluorescence of the antibody, it is possible to determine the amount of organic nitro compounds that are present and/or to decide whether organic nitro compounds are present.

By the use of this method and device for detection and analysis, it is possible to detect trace amounts of organic nitro compounds in samples of gas with greater sensitivity and speed than is possible with the conventional method and devices for detection and analysis, and the detection and analysis is simple, speedy, and specific.

### BRIEF DESCRIPTION OF THE DRAWINGS

This invention may be better understood and its numerous objects and advantages will become apparent to those skilled in the art by reference to the accompanying drawings as follows:

Figure 1 is a schematic diagram showing the main parts of a device for the detection of organic nitro compounds of this invention.

Figure 2 is a schematic diagram showing the structure of a 16-way valve that is one part of the device of Example 1.

Figure 3 is a graph of data obtained by measurement with the use of the device of Example 1.

Figure 4 is a graph of the results of measurement with the use of the device of Example 1.

### DESCRIPTION OF THE PREFERRED EMBODIMENTS

The method for the detection and analysis of organic nitro compounds of this invention will be explained here.

First, a sample gas is passed in the form of bubbles through a solvent, so that the components of the sample gas that are soluble in a solvent can be dissolved there, giving a sample solution. By this step of bubbling, any organic nitro compounds that are in the sample gas will go into solution in the solvent. As the solvent, all kinds of aqueous buffers, including pure water, can be used. Organic solvents, such as ethanol, methanol, acetone, and the like can be also used.

A solution of an antibody that specifically binds to the target substances (i.e., organic nitro compounds) to be detected is prepared by the dissolving of the antibody in a solvent. The solvent used here is preferably an aqueous buffer at the pH of 3 to 11 or else pure water, either of which will not interfere with the activity of the antibody; it is also possible to use a mixture of 50% ethanol and 50% methanol, or the like.

Next, the sample solution and the antibody solution are mixed together. Mixing can take place in a test tube or in an optical cell for use in the measurement of fluorescence intensity, but it is preferable that a vessel for mixing be used, if the assay is to be automated. As the device for mixing, it is possible to use, for example, a device for the preparation of concentration gradients for use in high-pressure liquid chromatography, magnetic stirrer, or a blender, but the use of a 16-way valve is preferred because dilution of the sample is prevented.

Next, the fluorescence intensity of the mixture of solutions is measured. The measurement of the fluorescence intensity is done by the use of an ordinary device for the measurement of fluorescence intensity. The shape of the optical cell for use in the measurement of the fluorescence intensity can be that of a batch cell, which is of a standard cell type, but for the sake of automation, a flow cell is preferred.

When measurement is to be automatic, pumps are needed for supply of the sample solution, the antibody solution, and the mixture of these two solutions. For the pumps, those for use in high-pressure liquid chromatography that allow accurate control of the flow rate, etc., are suitable for this purpose, but it is also possible to use peristaltic pumps and the like.

Organic nitro compounds that are the target substances for detection here include, as representative examples, the following: trinitrotoluene, dinitrotoluene, picric acid, tetryl, diazodinitrophenyl, etc., which are all aromatic nitro compounds. It is also possible to detect hexogen (cyclotrimethylenetrinitramine), octogen (cyclotetramethylenetetranitramine), nitroglycerine, nitroglycol, nitrocellulose, penthrite (pentaerythritol tetranitrate), and other nitro compounds. Also, it is, of course, possible to detect mixed powders and mixed explosives that contain these organic nitro compounds as components.

As the antibody, it is possible to use either polyclonal antibodies or monoclonal antibodies, provided that the antibody binds specifically to the target substance for detection (the organic nitro compounds) and provided that the fluorescence of the antibody decreases in intensity as the antibody binds to its antigen (i.e., organic nitro compounds). However, for the sake of reproducibility of the measurements, monoclonal antibodies that can be uniformly prepared in large amounts are preferable. As mentioned above, many of the antibodies that bind to organic nitro compounds show the phenomenon of fluorescence quenching.

It is possible to carry out the method for detection and analysis of this invention manually. However, as mentioned above, the antigen-antibody reaction occurs instantaneously, so the time taken by the assay is mainly the time needed to mix the sample solution and the antibody solution together, etc. Skill is needed if these steps are to be accomplished quickly. It is possible to improve workability and to shorten the time needed for measurement if a device is constructed that can carry out these steps automatically.

Below, this invention will be explained with reference to examples.

### Example 1

As the nitro compound to be assayed, trinitrotoluene (TNT), which is an aromatic nitro compound, was selected. TNT is a typical aromatic nitro compound, and one of the main components of many of the explosives manufactured at present.

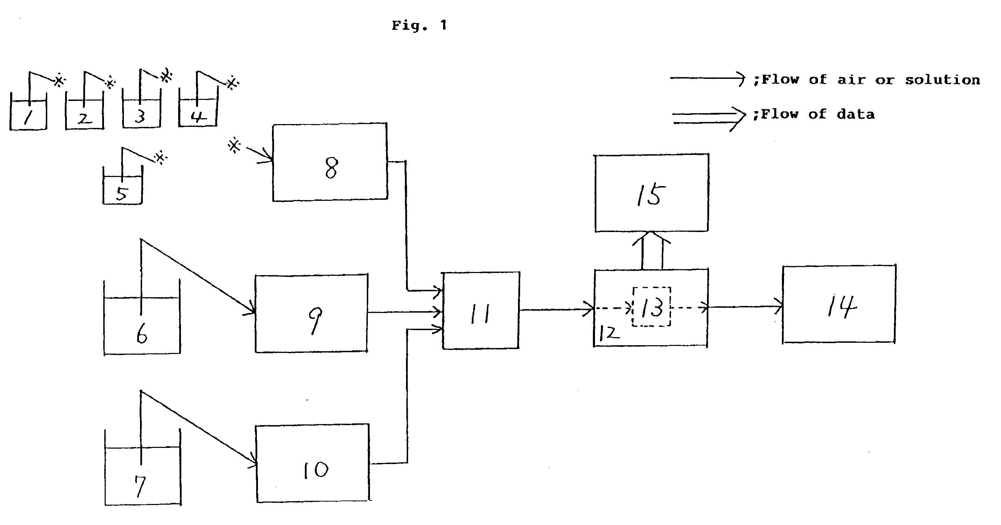

Figure 1 is a diagram showing a device of this invention used for measurement. Below, the method by which TNT in trace amounts in a gas was detected and the results of the measurements are explained.

### 1.1 Preparation of sample solutions 1-4 and reference sample solution 5:

(A) First, 1 g of TNT powder was left on the bottom of a three-mouthed volumetric flask with a capacity of 30ℓ, and the three openings thereof were sealed. The flask was left at 25°C for 24 hours.
(B) Sample gas was collected by the sampling of 12ℓ of the air in the flask by use of a glass syringe, with care taken so that the TNT powder would be undisturbed.
(C) Then, 10ℓ of the sample gas collected was bubbled through 1 ml of phosphate-buffered saline (PBS), pH 7.4, at the speed of 10ℓ per minute for 1 minute, and the PBS was used as sample solution 1.
(D) The sample gas that remained was diluted tenfold, 100-fold, and 1000-fold with air from an air cylinder that did not contain TNT.
(E) Then, 10ℓ of each of the sample gases diluted tenfold, 100-fold, and 1000-fold was treated with PBS as described above in C, giving sample solutions 2, 3, and 4.
(F) Separately, 10ℓ of air from the air cylinder that did not contain TNT was treated with PBS as described above in C, giving reference sample solution 5.
(G) Sample solutions 1, 2, 3, and 4 and reference sample solution 5 were assayed for their TNT concentration by use of an apparatus for gas chromatography.

The TNT concentration in sample solution 1 was about 1 x 10⁻⁸ g/ml, which is the lower limit of detection by the gas chromatograph. The concentrations of TNT in sample solutions 2, 3, and 4 and in reference sample solution 5 were below the limit of detection by the gas chromatograph.

The sample solutions 1, 2, 3, and 4 and the reference sample solution 5 prepared as described above were measured by the methods of this invention. The procedures used in this example were done by hand, but it would also have been easy, for example, to bubble the sample gas in the three-mouthed flask through the PBS automatically by the use of a pump or the like, thus preparing sample solution 1.

### 1.2 Preparation of antibody solution 6:

An anti-TNT antibody that has high binding affinity for TNT was dissolved in PBS at the concentration of 1 x 10⁻⁷ M, giving an anti-TNT antibody solution 6.

The antibody used could have been any antibody that bound with TNT specifically and that showed the phenomenon of fluorescence quenching when it bound to TNT. As such an antibody, there is, for example, the anti-TNT monoclonal antibody that is produced by the hybridoma 1-10-1 that was constructed from mouse myeloma cells (T. Mitsumata, J. Miyazaki, and H. Sugihara, Bioindustry Vol. 6, pp. 407-413, 1989). In this example, this monoclonal antibody was used.

### 1.3 Mixing each of the sample solutions 1-4 and the reference sample solution 5 with the antibody solution 6, and the measurement of the fluorescence intensity:

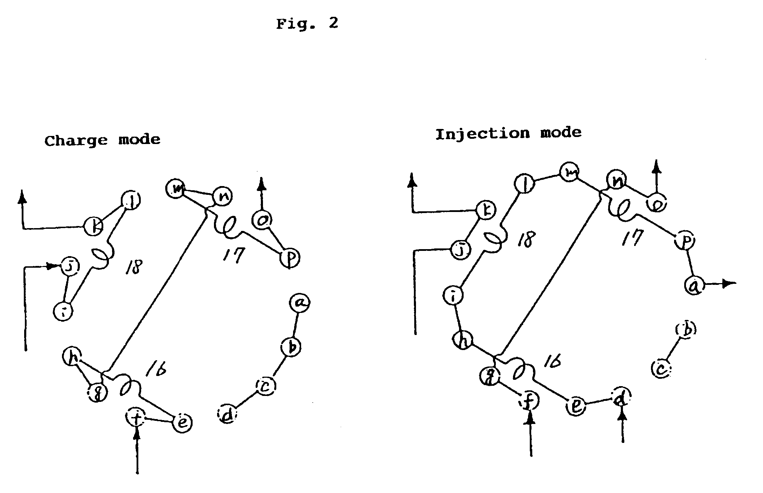

(A) Sample solution 1 was passed through a tube made of fluorine resin with an inner diameter of 1 mm by means of a pump 8 for high-pressure liquid chromatography, and introduced into a mixture vessel 11. The rate of flow was 1 ml/min and the time of flow was 10 seconds.
(B) In the same way as for sample solution 1 and under the same conditions, antibody solution 6 was introduced into mixture vessel 11 by means of a pump 9 for high-pressure liquid chromatography.
(C) In mixture vessel 11, the two solutions were mixed.
   Details of the construction of mixture vessel 11 are shown in Figure 2. Mixture vessel 11 was made with a 16-way valve, passage through which could be under two different conditions. The two modes of mixture vessel 11 were the charge mode and the injection mode, both shown in Figure 2. When sample solution 1 and the antibody solution 6 were introduced into mixture vessel 11, mixture vessel 11 was in the charge mode. Under these conditions, sample solution 1 was introduced through port f into the mixture vessel. Then the sample solution passed through ports e, h, g, n, m, p, and o, to reach a tube 14 for waste fluid. The space between port e and h and the space between ports m and p were provided with sample loops 16 and 17, respectively, each of which had the capacity of 100 µℓ. These were filled with the sample solution 1.
   Separately, antibody solution 6 passed through ports j, i, l, and k, in that order to reach the tub 14 for waste fluid. There was a sample loop 18 for the antibody solution between the ports i and l. The loop 18 had the capacity of 10 µℓ, and was filled with antibody solution 6.
   When the two solutions had been supplied to the mixture vessel 11, mixture vessel 11 was changed to the inject mode. At the same time, pump 10 for high-pressure liquid chromatography began to supply PBS at the flow rate of 4 ml/min to the port d. The PBS passed through the ports d, e, h, i, l, m, p, and a in that order to the flow cell 13, which was the optical cell in the fluorophotometer 12. At this time, the sample solution 1 in the sample loops 16 and 17 and the antibody solution 6 in the sample loop 18 were moved forward by pressure of the PBS. The antibody solution 6 was sandwiched between the two portions of sample solution 1, but while they were being introduced into the flow cell 13 in the fluorophotometer 12, the two solutions were mixed together by dispersion.
(D) As described above, the mixed solutions were introduced into the flow cell 13 in the fluorophotometer 12 at the flow rate of 4 ml/min by means of pump 10 for high-pressure liquid chromatography. The capacity of the flow cell 13 was 14 µℓ. The mixed solution that was introduced into flow cell 13 was then discarded into the tub 14 for waste fluid.
(E) While the solution in flow cell 13 was being irradiated with ultraviolet light at the wavelength of 280 nm, its fluorescence at 340 nm was continuously measured. The data from measurement were recorded with a pen recorder 15.
(F) By the same operations as were described above, sample solutions 2, 3, and 4 and reference sample solution 5 were assayed. The switching on and off the pumps 8, 9, and 10 for high-pressure liquid chromatography and the timing of the switching between modes of the mixture vessel 11 were all controlled by an externally housed computer with automatic control by control signals.

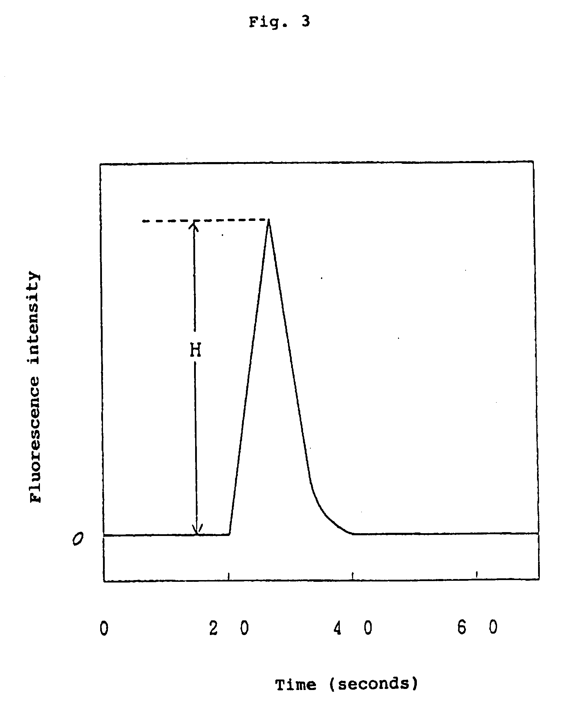

The examples of measurement obtained by the use of the device of Example 1 are plotted as a curve in Figure 3. The flow cell 13 was filled with PBS starting immediately after measurement began for the first 20 seconds, and so the fluorescence intensity was almost 0. At 20 seconds after measurement began, the sample solution 1 and antibody solution 6 began to reach the flow cell 13 as a mixed solution, and fluorescence arising from the antibody was observed. After this, the solution in the flow cell 13 was replaced with PBS, and the fluorescence intensity once more was about 0 starting at about 40 seconds after the start of measurement. Thus, the time needed for one measurement was 40 seconds or less. The fluorescence intensity and the height H of the peak for the mixed solution were related proportionally, as in ordinary flow injection analysis.

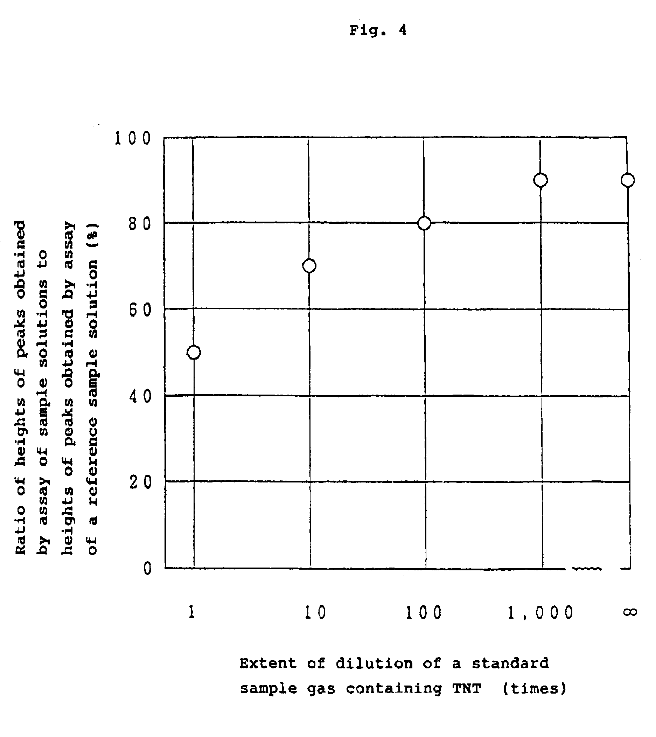

Next, the heights H of the peaks obtained during the assay of sample solutions 1, 2, 3, and 4 are shown in Figure 4 as percentages of the height H of the peak obtained during the assay of reference sample solution 5.

The heights of the peaks obtained by the assay of the sample solution 1 used as is, with no dilution, and the sample solutions 2 and 3, diluted tenfold and 100-fold, respectively, were smaller than the height of the peak obtained for reference sample solution 5. The reason, as was mentioned before, is that the TNT in sample solutions 1, 2 and 3 bound with the anti-TNT monoclonal antibody in the antibody solution 6, and for that reason, the fluorescence of the anti-TNT monoclonal antibody decreased. Also, as mentioned in step G of 1.1 of Example 1, when a gas chromatograph was used for the measurement, it was not possible to detect TNT in the sample solutions 2, 3, or 4. The time needed for a single measurement with the use of the device in Example 1 was 40 seconds, and that needed with the use of the gas chromatograph was 20 minutes.

The results given above show that by the use of the device of Example 1, trace amounts of TNT that could not be detected by a gas chromatograph could be detected in about two minutes, including the time needed for collection of the gas.

In Example 1, an anti-TNT monoclonal antibody was used as an antibody, but it is also possible to detect TNT by the use of an anti-TNT polyclonal antibody.

TNT was used here as the example of an organic nitro compound to be detected in Example 1, but it is possible to detect other organic nitro compounds in the same way as was described above.

Next, an organic nitro compound that is not an aromatic nitro compound will be used as the compound to be detected and the antibody used will be a polyclonal antibody, with measurement being done manually, in the following Example 2.

### Example 2

The organic nitro compound to be detected was nitroglycerine (NG). NG is a main component of dynamite, and is a typical organic nitro compound that is not aromatic.

### 2.1 Preparation of sample solution, reference sample solution, and antibody solution:

(A) First, 1 g of NG was left on the bottom of a three mouthed volumetric flask with a capacity of 30ℓ, and the openings of the flask were sealed. The flask was then left for 24 hours at 25°C.
(B) Then, 10ℓ of air was collected from the flask with the use of a glass syringe, and used as a sample gas.
(C) The 10ℓ of the sample gas that was collected was bubbled through 1 ml of PBS, pH 7.4, at the rate of 10ℓ per minute, for one minute, giving the sample solution.
(D) Separately, 10ℓ of air from a gas cylinder that did not contain NG was treated by the procedure in C, giving a reference sample solution.
(E) By the same method as in Example 1, PBS was used to prepare an anti-NG polyclonal antibody solution with the concentration of 1 x 10⁻⁷ M.

### 2.2 Mixing of the sample solution and the reference sample solution separately with the antibody solution and the measurement of the fluorescence intensity thereof:

(A) First, 760 µℓ of the antibody solution prepared in 2.1 was introduced into a standard cell for the measurement of fluorescence.
(B) Then, 40 µℓ of the sample solution was added to the antibody solution, mixed well by means of a glass stick, and used as a mixed solution.
(C) The fluorescence intensity of the mixed solution was measured by the use of a fluorophotometer. The excitation wavelength was 280 nm, and the measurement of fluorescence was at the wavelength of 340 nm. The width of the slit for the excitation wavelength was 5 nm, and that for the fluorescence wavelength was also 5 nm.
(D) The reference sample solution was measured in the same way for fluorescence intensity.

When the reference sample solution was used, the fluorescence intensity of the mixed solution was 40. The fluorescence intensity when the sample solution was used instead of the reference solution in the mixed solution was 20. The decrease in fluorescence intensity was 50%. Thus, NG could be detected. The time needed for a single measurement was about 10 minutes.

## Claims

1. A method for the detection and analysis of organic nitro compounds comprising mixing a sample solution with a solution of antibody that binds specifically to said organic nitro compounds dissolved in solvent so as to prepare a mixed solution, and measuring the fluorescence intensity of said mixed solution to thereby detect the presence and/or the amount of said organic nitro compounds.

2. A method for the detection and analysis according to claim 1, wherein the mixture of the sample solution and of the antibody solution to prepare a mixed solution and also the measurement of the fluorescence of said mixed solution are carried out continuously in a flow system.

3. A method for the detection and analysis according to claim 1 or 2, wherein a sample gas is dissolved in solvent for the preparation of said sample solution.

4. A method for the detection and analysis according to claim 1 or 2, wherein said antibody is a monoclonal antibody.

5. A method for the detection and analysis according to claim 1 or 2, wherein said organic nitro compound to be detected is an aromatic nitro compound.

6. A method for the detection and analysis according to claim 1 or 2, wherein said organic nitro compound to be detected is one selected from the group consisting of trinitrotoluene (TNT), hexogen, octogen, nitroglycerin, nitrocellulose, nitroglycol, and penthrite.

## Patentansprüche

1. Verfahren zum Nachweis und zur Analyse organischer Nitroverbindungen, bei dem man eine Probelösung mit einer spezifisch an die in einem Lösungsmittel aufgelösten organischen Nitroverbindungen bindenden Antikörperlösung vermischt, um eine Mischlösung herzustellen, und die Intensität der Fluoreszenz dieser Mischlösung mißt, um dadurch das Vorhandensein und/oder die Menge der organischen Nitroverbindungen nachzuweisen.

2. Verfahren zum Nachweis und zur Analyse nach Anspruch 1, bei dem die Vermischung der Probelösung und der Antikörperlösung zur Herstellung einer Mischlösung sowie die Messung der Fluoreszenz der Mischlösung kontinuierlich in einem Strömungssystem durchgeführt werden.

3. Verfahren zum Nachweis und zur Analyse nach Anspruch 1 oder 2, bei dem zur Herstellung der Probelösung ein Probegas in einem Lösungsmittel aufgelöst wird.

4. Verfahren zum Nachweis und zur Analyse nach Anspruch 1 oder 2, bei dem der Antikörper ein monoklonaler Antikörper ist.

5. Verfahren zum Nachweis und zur Analyse nach Anspruch 1 oder 2, bei dem die nachzuweisende Nitroverbindung eine aromatische Nitroverbindung ist.

6. Verfahren zum Nachweis und zur Analyse nach Anspruch 1 oder 2, bei dem die nachzuweisende organische Verbindung aus der aus Trinitrotoluol (TNT), Hexogen, Octogen, Nitroglycerin, Nitrocellulose, Nitroglykol und Pentaerythrit-tetranitrat bestehenden Gruppe ausgewählt ist.

## Revendications

1. Méthode pour la détection et l'analyse de composés nitro-organiques comprenant le mélange d'une solution d'échantillon avec une solution d'un anticorps qui se lie spécifiquement auxdits composés nitro-organiques dissous dans un solvant de façon à préparer une solution de mélange, et la mesure de l'intensité de fluorescence de ladite solution de mélange pour y détecter la présence et/ou la quantité desdits composés nitro-organiques.

2. Méthode pour la détection et l'analyse selon la revendication 1, dans laquelle le mélange de la solution d'échantillon et de la solution d'anticorps pour préparer une solution de mélange et également la mesure de fluorescence de ladite solution de mélange sont réalisés dans un système à flux continu.

3. Méthode pour la détection et l'analyse selon la revendication 1 ou 2, dans laquelle un échantillon de gaz est dissous dans un solvant pour la préparation de ladite solution d'échantillon.

4. Méthode pour la détection et l'analyse selon la revendication 1 ou 2, dans laquelle ledit anticorps est un anticorps monoclonal.

5. Méthode pour la détection et l'analyse selon la revendication 1 ou 2, dans laquelle ledit composé nitro-organique à détecter est un composé nitro-aromatique.

6. Méthode pour la détection et l'analyse selon la revendication 1 ou 2, dans laquelle ledit composé nitro-organique à détecter est l'un de ceux choisis dans le groupe constitué par trinitrotoluène (TNT), hexogène, octogène, nitroglycérine, nitrocellulose, nitroglycol et penthrite.
